# EUROPEAN PATENT SPECIFICATION

(11) **EP 0 929 272 B1**
(45) Date of publication and mention of the grant of the patent: **28.11.2001**
(21) Application number: 97939971.4
(22) Date of filing: 05.09.1997
(51) Int. Cl.: A61F 2/06

(54) **AN AGGREGATE FOR TRANSLUMINAL INSERTION OF A TUBULAR STENT, AND AN ENDOVASCULAR GRAFT DEVICE**
EINRICHTUNG ZUR TRANSLUMINALEN EINFÜHRUNG EINES TUBULAREN STENTS UND EINE ENDOVASKULÄRE TRANSPLANTAT-VORRICHTUNG
ENSEMBLE D'INSERTION TRANSLUMINALE D'EXTENSEUR TUBULAIRE ET DISPOSITIF DE GREFFE ENDOVASCULAIRE

(30) Priority: 06.09.1996 DK 96196
(43) Date of publication of application: 21.07.1999
(73) Proprietor: William Cook Europe ApS, 4632 Bjaeverskov (DK)
(72) Inventor: KAVTELADZE, Zaza A., Moscow, 117330 (RU); KORSHOK, Alexandr P., Moskovskaya obl., Moscow, 130070 (RU)
(74) Representative: Indahl, Peter Jensen
(86) International application number: DK9700370
(87) International publication number: WO9809583

(56) References cited:
- EP-A- 0 539 237
- US-A- 5 366 473

## Description

The present invention relates to an aggregate for transluminal insertion of a tubular stent which, in its placed condition in a vessel in the vascular system, is provided at its periphery with a tubular coating along at least part of its length, which aggregate includes an introducer sheath with a stent arranged inside the sheath in a first condition with a reduced diameter, and an extruder device which can be manipulated to release the stent from the introducer sheath, the stent being adapted to expand to a second condition with a larger diameter.

Such an aggregate is known from EP-A-0 539 237. This prior-art aggregate is intended for use in the repair of an abdominal aortic aneurysm, where a graft consisting of a stent and a blood-impermeable tubular coating arranged at the periphery of the stent is inserted in the aorta at the aneurysm and placed in such a manner that the graft cuts off the aneurysm from the bloodstream in the aorta. A number of other stents provided with a tubular coating are also known, *vide,* for example, WO95/05132, in which the stent is placed in or permanently connected with the tubular material, WO93/22986, in which a stent for placement in the oesophagus is provided with a tubular coating at the stent middle, but the stent ends are uncoated, and the publications EP-A-0 696 446 and WO94/24961, in which the stent is embedded in the tubular material.

The prior-art stents with a tubular coating suffer from the disadvantage that they are inserted by means of an introducer sheath of a relatively large external diameter, typically from 6.0 to 8.7 mm (18-26 French). This necessitates femoral insertion and surgical incision at the site of insertion to provide space for passage of the introducer sheath into the vascular system.

The object of the present invention is to provide an aggregate rendering possible true percutaneous insertion of a stent and a tubular coating by means of the Seldinger technique.

In view of this, the above aggregate is characterised in that in that the stent is arranged in its first condition at least partially inside the introducer sheath in direct contact with the inner sheath surface, and that at least the major part of the tubular coating is positioned separate from the periphery of the stent while the stent is in its first condition.

With this arrangement of the tubular coating in connection with the aggregate, the coating is not placed on the periphery of the stent until during the release of the stent from the introducer sheath by the expansion from the first to the second condition.

By mounting the tubular coating in the aggregate in such a manner that it is not placed directly on the outer surface of the stent, the stent can be arranged in direct contact with the inner surface of the introducer sheath. This means that the internal diameter of the introducer sheath can be made smaller than in prior art, partly because space is not required for both the body material of the stent and for the coating at the same place inside the sheath, partly because the body material of the stent, typically a filamental metallic material, can more easily be packed into a compact configuration when the stent material is free of the tubular coating. With this design of the aggregate it is possible to arrange the stent inside an introducer sheath having an external diameter of about 5.3 mm (16 French) or less, typically ranging from 2.7 to 5.3 mm (8-16 French).

The aggregate according to the invention renders possible insertion by other routes than the femoral one; the percutaneous insertion may, for example, be effected axillarily (at the armpit), radially (through the arm), cervically (at the throat) or elsewhere having a vessel of medium or large size located close to the skin. The largest introducer sheath that can be used with the Seldinger technique has an external diameter of 16 French. In addition to rendering possible other access routes than the femoral one, the invention also provides advantages in case of femoral percutaneous insertion. The puncture hole at the site of insertion is substantially smaller than with the known techniques, which is partly less traumatic to the patient, partly produces less bleeding out of the puncture hole at replacement of introducer sheath, catheters, etc. The loading method of the stent into the introducer sheath is thus of substantially less importance than in prior art, *vide* the mention of "muzzle loading" as opposed to "breech loading" in EP-A-0 539 237.

In one embodiment the tubular coating may at some positions be connected with the stent, preferably at the place on the stent length being at or closest to the distal end of the introducer sheath. The connection between the stent and the coating provides the advantage that the coating is placed on the periphery of the stent in a predetermined position along the length of the stent when the stent is placed at the desired site. The connection will typically have been made at one end of the coating, and it is normally sufficient that the stent is provided with a single-layer coating. However, it is possible to effect the connection at another location on the coating, such as at the middle thereof, the result of which is that at least along part of its coated length the stent becomes provided with a two-layer coating. The coating may be connected with the distal end of the stent or be connected with the stent at a distance therefrom proximally to this end. In this connection the distal end means the leading end during the insertion, and the proximal end means the following end inserted as the last one. If the coating is connected at a distance from the distal end of the stent, the stent portion distally from the connection must be kept in the first position by a member being separate from the introducer sheath so that the coating may pass radially in between the sheath and the member and thereby reach the fastening location. Preferably the coating is connected with the stent at points or along a circular path located in a plane at right angles to the longitudinal axis of the stent. The connection may, for example, be effected by means of suture, glue, engagement means embedded in the coating or by the coating being shaped around one or more pieces of material in the stent body.

In an alternative embodiment the aggregate keeps the tubular coating completely separated from the stent until the release of the latter has commenced. The coating is thus not fastened to the stent. This provides the advantage that no separate fastening means, such as suture or glue, have been used that may act as foreign bodies in the body after the placement and that may further take up space while the stent is inside the introducer sheath in its first condition.

In a preferred embodiment of the aggregate, the tubular coating surrounds the introducer sheath. After the placement, the coating has a diameter corresponding to the diameter of the stent in its second, expanded condition. Most frequently, the coating is not very elastic or substantially inelastic, and this means that the coating has a circumferential length substantially larger than the circumferential length of the inner surface of the introducer sheath. By arranging the coating around the introducer sheath, the outer surface of which has a larger circumferential length than the inner surface, the coating need not be folded or bent as much together as at an arrangement inside the sheath. The external arrangement therefore facilitates the spreading of the coating at the expansion of the stent to a larger diameter. With the external location it is also possible to mount the coating in the aggregate so that in the longitudinal direction it is extended over a length which is not smaller than the coated length on the outer surface of the stent after the placement. Folding of the coating in the longitudinal direction is thus avoided, which contributes to the coating only causing a relatively small diameter increase for the total aggregate. A further advantage is that the stent is only put in touch with the inner surface of the coating when the stent begins expanding to a larger diameter after its extrusion through the distal end opening of the introducer sheath. Thus, the introducer sheath contributes to an orderly placing of the coating on the outer stent surface.

The aggregate may comprise a catheter surrounding the tubular coating and the introducer sheath until the aggregate has been transluminally advanced to a location close to the site of placement. The catheter protects the coating during the advancing from the puncture hole to the area near the site of placement where the introducer sheath is extruded through the distal opening of the catheter before placement of the stent is commenced. It is possible to advance the catheter in the vessel before the introducer sheath with the coating and the stent is inserted into the patient, but normally the whole aggregate is preferably inserted at the same time because this renders it possible to advance the introducer sheath through the catheter in advance and under orderly circumstances while the catheter assumes a straight shape. If the catheter is to be advanced in the vascular system along a route having considerable curves for positioning at a site of placement with a small vessel lumen, it may, however, be advantageous first to insert the catheter together with a guidewire and then to retract the guidewire from the patient, whereupon the introducer sheath is pushed forward through the catheter. In this case the inner surface of the catheter may be formed in a manner reducing friction between the sheath and the inner catheter surface. Such a separate advancing of the introducer sheath renders it possible to place a stent coating at a site of a very small vessel lumen and a correspondingly small diameter of the stent in its second, expanded condition.

In one embodiment the aggregate comprises an annular cavity in which the tubular coating lies protected until release of the stent. The annular cavity may be formed by the outer surface of the introducer sheath and the inner surface of a catheter, viz., corresponding to the embodiment just mentioned. Another possibility is to form the annular cavity with an outer wall which surrounds the tubular coating and has an external diameter substantially corresponding to the external diameter of the introducer sheath. Thus the whole aggregate may have an external diameter which corresponds to the sheath and is smaller than the outer diameter of said catheter.

In a preferred embodiment the cavity is formed in the distal end of the introducer sheath, preferably by the sheath at this end having an elongated recess shielded inwards by means of a tube of a material which is flexible and substantially unstretchable. As the inner shielding tube is made of an unstretchable material it can absorb the radial pressure from the stent loaded into the introducer sheath as hoop stresses in the tube material so that the radial pressure from the stent does not affect the tubular coating which has been received in the annular space between the shielding tube and inner surface of the introducer sheath. At the same time the shielding tube may, by suitable choice of its material, be bending flexible so that the distal end of the aggregate is suitably soft to facilitate advancement through the vessels and avoid damage to the vessel walls. The recess and thus the annular space may have a suitably large size in the radial direction so that the coating may be arranged in a bellows-shaped pattern in the recess, whereby the recess achieves shorter length. Preferably, however, the recess has a length corresponding to the length of the coating, and a radial depth suitably small so that the coating can just be received in the recess, the aggregate thus obtaining an advantageously small external diameter.

If it is not desired to provide the stent with a coating in one or both end sections so as to obtain a stronger fixation of the stent ends to the vessel in which the placement is effected, the stent is preferably arranged around the extruder device extending centrally through the introducer sheath, the stent in its first condition being located partially in the distal section of the introducer sheath, partially in an annular cavity in the head of the extruder device, and the distal end of the tubular coating being arranged proximally at a distance from the distal end of the stent. When the stent is placed, the distal end section of the stent lying radially compressed in the head of the extruder device during the insertion may expand for fixation against the vessel wall without being provided with coating. At the proximal end of the stent, a coating-free end section can be established by the coating being suitably shorter than the stent body in its second, radially expanding condition.

The stent may be formed so that it is self-expanding and largely does not change its length at the expansion. Stents of this type are applicable in connection with the invention. Alternatively the stent may be formed so that its length is reduced at the expansion from the first to the second condition. This means that after engagement with the inner surface of the tubular coating an expanding stent area obtains a shorter length at the continued expansion. Thus, the stent is prevented from affecting the coating with longitudinal tensile stresses. On the contrary, the stent may contract the coating somewhat, which cannot overload the material of the coating. At the same time the coating is not able to lock the expanding stent material in an intermediate position without full expansion.

It may be an advantage when the tubular coating is arranged in a folded condition around the introducer sheath that the surfaces facing the introducer sheath are at least partially in contact with an adhesive. The adhesive retains the coating on the outer surface of the introducer sheath until the stent begins to spread out the coating at the start of the expansion, which breaks the bond produced by the adhesive between the coating and the sheath. Preferably, the adhesive is arranged as far as possible on the outer sheath surface so that after placement of the stent the sheath has no substantial amount of adhesive left.

The present invention further relates to an endovascular graft device comprising at least a first stent for fixation against a vessel wall in the vascular system and a second stent for placement fully or partially inside the first stent as well as a tubular coating, said first and said second stent having a first condition with a reduced diameter and a expanded second condition with a larger diameter. Such a graft device is known from EP-A-0 551 179 for repair of an abdominal aortic aneurysm. The first stent is here placed on the infrarenal aorta portion upstream of the aneurysm, whereupon the cranial ends of two graft devices with permanent coatings on the stents are balloon expanded for fixation with their cranial end openings located on a level with the cranial end opening in the first stent. The first stent serves to protect the aorta wall against the relatively large, radially outward pressure influences occurring when the two grafts are balloon expanded. Of necessity, these forces have to be large to deform the caudal ends of the two grafts to cover the circular cross-section in the first stent.

The object of the graft device according to the invention is to anchor a stent with a tubular coating at a site where the vessel wall does not have sufficient extent to provide a safe mounting of a coated stent graft. In view of this the graft device is characterised in that at least the major part of the tubular coating is positioned in an aggregate separate from the periphery of the second stent while this stent is in said first condition, that in said second condition the first and the second stents have substantially the same diameter, and that after placement of the first and second stents in said second condition the tubular coating is at least partially fixed between the inner surface of the first stent and the outer surface of the second stent. In addition to achieving said insertion advantages, the coating is fixed very securely by the squeezing between the two stent bodies. This is an advantage, for example, if the coating hangs down past the caudal end of the second stent, which may be the case when the coating forms a trouser-shaped graft body with two lower openings each provided with a separate graft body, as described by the Applicant in WO95/16406.

Another advantage of the graft device is that the first stent may be formed as a relatively open stent of a heavy material and may be placed so that it extends from the top and some way down past the renal arteries, whereupon the second stent may be placed inside the first one in a position where the coating is arranged infrarenally. This permits the bloodstream to the renal arteries to pass the graft device without being hindered by the coating, and at the same time the second stent is supported by the caudal end section of the first stent, which is securely fixed to the aorta wall suprarenally. Such a graft device is especially suitable for repair of abdominal aortic aneurysms in which the infrarenal aorta portion upstream of the aneurysm is very short.

Examples of embodiments of the invention will now be described in further detail below with reference to the drawing, in which
Figs. 1-6 show longitudinal sections through the distal ends of six different embodiments of the aggregate according to the invention,
Figs. 7 and 8 are two different stages in the placement of the stent from the embodiment shown in Fig. 6,
Figs. 9-12 show four different stages of the insertion and release of the stent in a seventh embodiment of the aggregate,
Figs. 13 and 14 are cross-sectional views of transverse folds in a tubular coating,
Fig. 15 is an illustration of a graft device according to the invention positioned at an abdominal aortic aneurysm, and
Fig. 16 is a cross-sectional view through an introducer sheath with an annular cavity in which a coating is positioned.

In the subsequent description of the different embodiments the same designations of the main elements of the invention are used for the sake of simplicity. The main elements of the aggregate are a tubular stent 1, a tubular coating 2, an introducer sheath 3 and an extruder device 4. In some embodiments the aggregate may also comprise a catheter 5.

The stent 1 may have many different designs. It may, for example, be constructed from sets of wires which extend in a spiral shape with opposite directions of windings through the stent body, as described in German patent No. 33 42 798, or as described in US-A-5 370 683 be formed by a single wire bent in suitable courses and laid in a spiral course, or as described in EP-A-0 645 125 be made of a wire bent and arranged in a course forming rhomboid cells, or be of the so-called Gianturco Z-stent type, or be formed as described in DE-A-39 18 736 or in WO94/03127. The stent may further be formed by several wires bent together to form heart-shaped or arrow-shaped cells where the wires are wound about each other at the points of intersection or are knotted to form knots as partially described in the Applicant's WO 97/09945 (corresponding to Danish patent application No. 995/95, which was made available to the public on 8 February 1996). With reference to the latter application the description therein of various stent designs is hereby incorporated into the present application.

The stent 1 is expandable from a first condition with a reduced diameter to a second condition with a larger diameter. For the fields of application mentioned in the following for the stent, ranges are mentioned for the typical stent diameter when assuming its second condition: coronary 2-4 mm; iliac, femoral and renal 6-12 mm; carotid 6-12 mm; aortic aneurysm 15-30 mm; vena cava 12-30 mm; vena subclavia 12-30 mm; arteriovenous shunt endoprosthesis 6-14 mm; TIPS (bypass in liver) 10-12 mm; ureter 4-7 mm; urethra 4-7 mm; oesophagus 18 mm at the middle; biliary 6-10 mm; pancreas 2-3 mm; and bronchial 15-20 mm. The stent material may be stainless steel, nitinol, titanium, tantalum, a copper alloy or a plastic material such as modified butadiene.

The tubular coating 2 may be of polyester (such as Dacron TM), polytetrafluoroethylene PTFE, polyurethane PU, polyethylene PE, propylene PP, nylon, carbon fibres or any other biocompatible material capable of blocking a bloodstream or another flow of body fluid. The coating may be of a foil which is completely impermeable, but the coating is preferably porous to promote fibrous ingrowth and thus anchoring of the coating in the body vessel in question. The porosity may be produced by arranging salt grains of a predetermined sieve dimension in the synthetic material and wash out the salt grains after establishment of the foil shape. The coating may also be made by weaving threads into a tubular body. Another possibility is to manufacture the foil from a porous expanded PTFE.

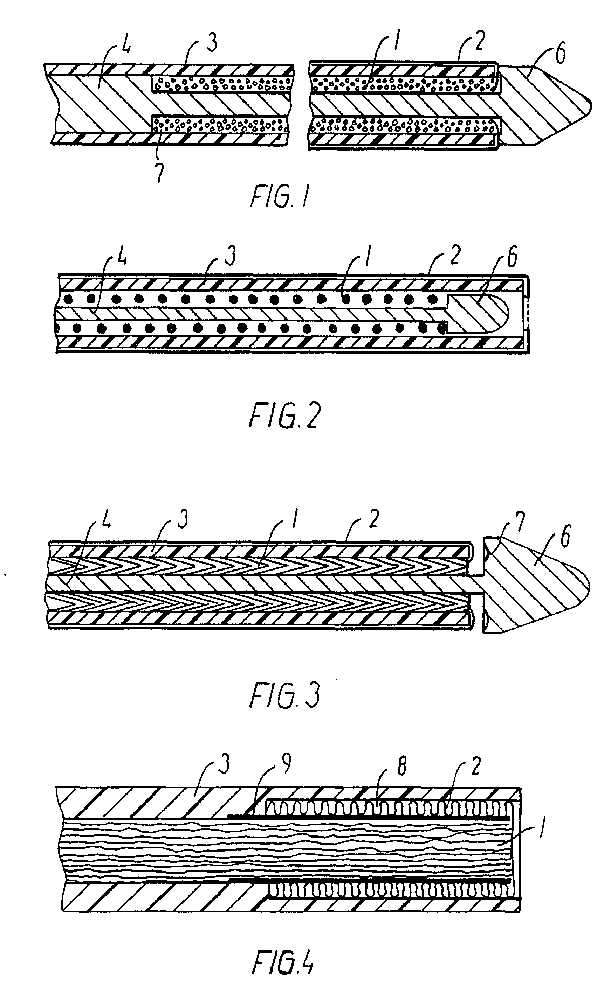

Fig. 1 shows the stent 1 arranged in its first condition with a reduced diameter inside the introducer sheath 3. The stent is received in a recess in the extruder device 4 which at the distal end has a head 6 lying protectively in front of the distal opening of the sheath. The rounded front end of the head facilitates the advancement to the desired place or the pushing into a catheter, if such is used. If desired, the head 6 may be provided with a distally projecting soft guidewire portion. The annular proximal end wall 7 in the recess pushes at the proximal end of the stent when the device 4 is moved forwards in relation to the sheath 3 or the sheath is pulled back. In addition, at axially separated places in the recess the device 4 may have projecting engagement members supporting the stent during the placement. The foil or coating 2 is connected to the distal end of the stent and extends from the place of connection radially out between the distal end of the sheath and the backward end surface of the head 6. On the outer surface of the sheath the foil has been turned back to a longitudinally elongated position in contact with the sheath.

The second embodiment shown in Fig. 2 deviates from the first one in that the head 6 has a sufficiently small diameter to pass through the inner lumen of the sheath, and that the foil 2 is completely separated from the stent 1. This provides an extra possibility of variation at the insertion, viz., that the introducer sheath with the foil 2 is first inserted to the desired site, whereupon the device 4 with the stent is pushed forward and placed. The distal end of the foil projects down about the distal opening of the sheath so that the rim of the foil is gripped by the outer surface of the stent at the beginning expansion of the stent when it is extruded through the distal opening.

The third embodiment in Fig. 3 shows a stent 1 with heart or arrow-tip-like lattice cells inserted in the sheath 3. Such a stent can be compressed to a very small diameter in the first condition as seen in relation to the diameter in the second condition. The foil 2 is connected with the stent end, and at the annular rim of the sheath 3 the head 6 has an annular recess 7 lying protectively around the foil end during the insertion.

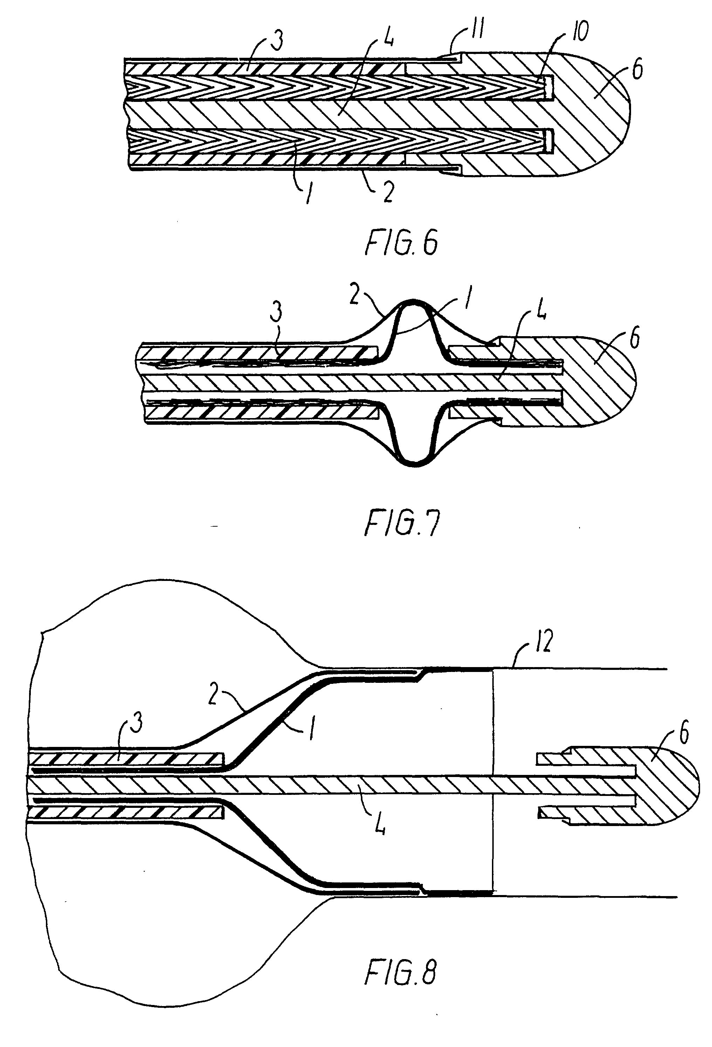

The fourth embodiment shown in Fig. 4 is of a type in which the foil 2 is received inside an annular cavity 8 produced by the sheath 3 having at its distal end section an elongated recess which is inwardly shielded by means of a tube 9 of a material being flexible and substantially unstretchable. The tube 9 may, for example, be made of PTFE, of carbon fibre, of dacron or of mylar. Viewed in a longitudinal section, the foil 2 is arranged in a bellows-shaped pattern and the foil is pulled out of the distal annular opening of the recess when the stent is extruded. Fig. 5 shows a similar design in which the cavity 8 is so long that the foil can lie full-length in its longitudinal direction. The foil may, for example, be pre-mounted on the outer surface of the tube 9 before the latter is mounted in the sheath 3.

Fig. 6 shows a sixth embodiment in which the stent is arranged around the extruder device 4, which extends centrally through the introducer sheath 3. The stent is located partially in the distal section of the introducer sheath, partially in an annular cavity 10 in the head 6 of the extruder device. The distal end of the tubular coating 2 is located proximally at a distance from the distal end of the stent, and the coating is arranged on the outer surface of the sheath completely separated from the stent 1. The distal end of the coating lies inserted under a circumferential spring flap 11 on the outside of the head so that the coating retains its longitudinal location during the insertion to the desired site of placement.

Fig. 7 shows the beginning release of the stent at the site of placement. The release is effected by the sheath being moved backwards in relation to the head 6. The released area of the stent 1 expands to a larger diameter and thus catches the coating 2. Then the head 6 can be moved away from the stent by the device 4 being pushed forwards so that the distal end of the stent expands to its full diameter into engagement with the vessel wall 12 (see Fig. 8), whereupon the sheath 3 can be retracted from the patient. It can be seen that the foil 2 does not cover the distal end section of the stent. Alternatively, it is possible to fasten the foil to the outer surface of the stent, the foil then in the mounted position in the aggregate passing into the space between the back of the head 6 and the front of the sheath 3.

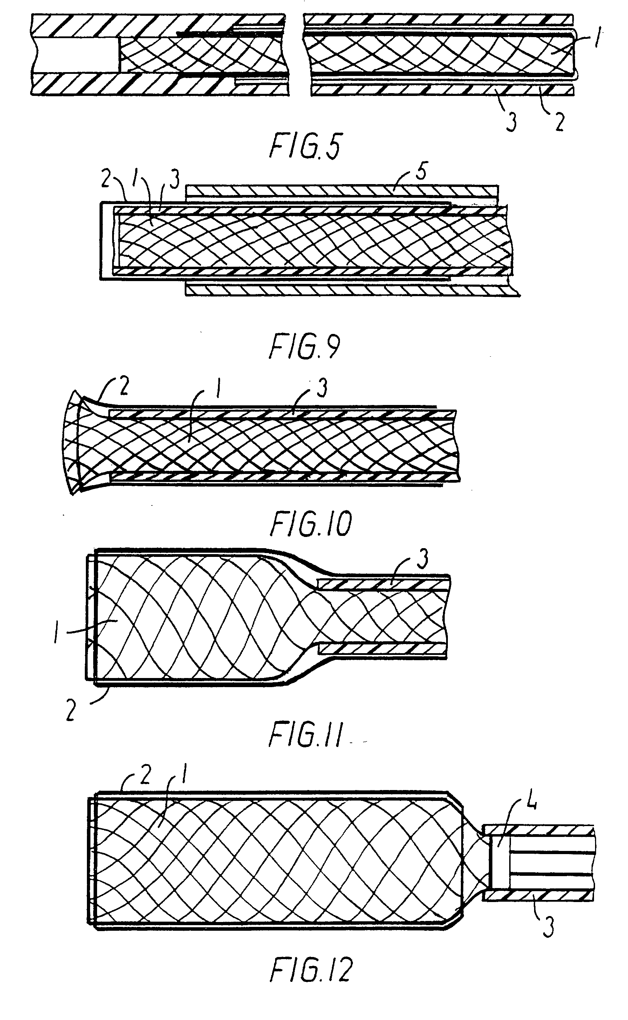

Fig. 9 shows the stent 1 inside the sheath 3 and the foil 2 on the outer surface thereof. The sheath is arranged in a catheter 5, and the subsequent Figures 10 to 12 show the extrusion of the stent 1 from the sheath with simultaneous fastening of the foil to the outer stent surface. The stent is extruded from the sheath 3 by means of an extruder device 4 in the form of a pusher.

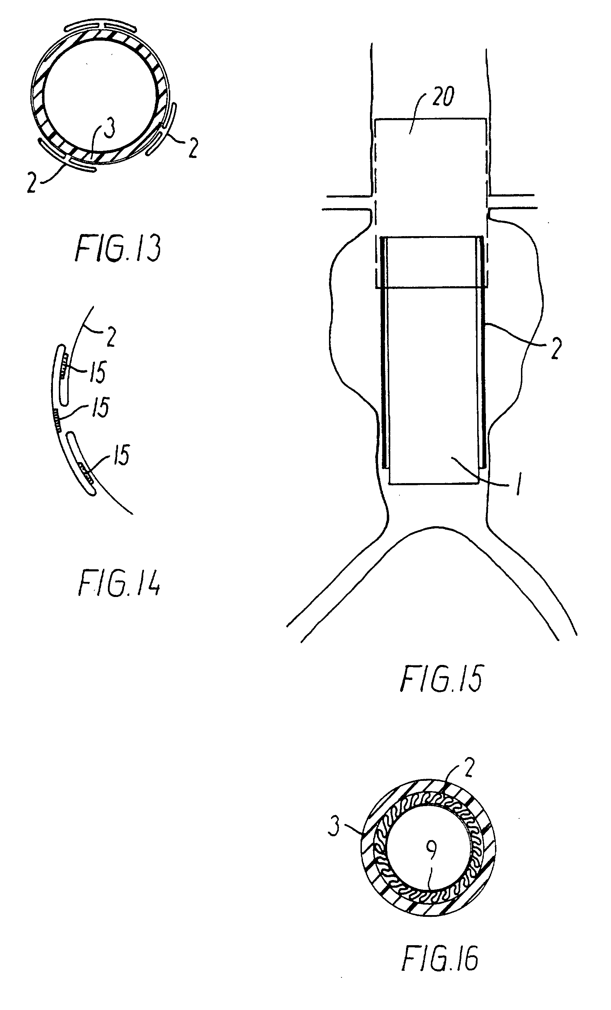

Fig. 13 shows how the foil 2 can be folded on the outer surface of the sheath 3. Fig. 14 shows that the foil can be temporarily fastened to the outer sheath surface by means of an adhesive 15. The folding allows for a rather great length of foil or covering to be received in little space.

Fig. 16 shows that the foil 2 can be received between the sheath 3 and the tube 9 folded in a wave-shaped pattern in the circumferential direction. This embodiment is particularly advantageous when the foil 2 is positioned in a straightened longitudinal position, as shown in Fig. 5.

In Fig. 15, a first stent 20 is seen to be placed at the renal arteries, and the stent 1 is here a second stent provided with the coating foil 2, which cuts off an aneurysm from the aorta. The first stent 20 can be relatively open at least in the areas opposite the renal arteries so that the renal blood flows are unhindered. The second stent is placed infrarenally inside the first one and the coating foil 2 is thereby fixedly locked in between the two stents in the area of their overlap.

The various embodiments described above can be combined to new embodiments within the present invention. Further, in the present context coating or foil includes any kind of covering or graft material to be positioned on the stent.

## Claims

1. An aggregate for transluminal insertion of a tubular stent which, in its placed condition in a vessel in the vascular system, is provided at its periphery with a tubular coating along at least part of its length, which aggregate includes an introducer sheath (3) with a stent (1) arranged inside the sheath in a first condition with a reduced diameter, and an extruder device (4) which can be manipulated to release the stent from the introducer sheath, the stent being adapted to expand to a second condition with a larger diameter, **characterized in that** the stent (1) is arranged in its first condition at least partially inside the introducer sheath (3) in direct contact with the inner sheath surface, and that at least the major part of the tubular coating (2) is positioned separate from the periphery of the stent while the stent is in its first condition.

2. An aggregate according to claim 1, **characterized in that** the tubular coating (2) is connected with the stent (1), preferably at the place on the stent length being at or closest to the distal end of the introducer sheath (3).

3. An aggregate according to claim 1, **characterized in that** the aggregate keeps the tubular coating (2) completely separated from the stent (1) until the release of the latter has commenced.

4. An aggregate according to any one of claims 1-3, **characterized in that** the tubular coating (2) surrounds the introducer sheath (3).

5. An aggregate according to claim 4, **characterized in that** the aggregate comprises a catheter (5) surrounding the tubular coating (2) and the introducer sheath (3), preferably until the aggregate has been transluminally advanced to a location close to the site of placement.

6. An aggregate according to any one of claims 1-5, **characterized in that** the aggregate comprises an annular cavity (8, 10) in which the tubular coating (2) lies protected until release of the stent.

7. An aggregate according to claim 6, **characterized in that** the cavity (8) is formed in the distal end of the introducer sheath (3), preferably by the sheath at this end having an elongated recess shielded inwards by means of a tube (9) of a material which is flexible and substantially unstretchable.

8. An aggregate according to any one of claims 1-7, **characterized in that** the stent (1) is arranged around the extruder device (4) extending centrally through the introducer sheath (3), that the stent in its first condition is located partially in the distal section of the introducer sheath, partially in an annular cavity (10) in the head of the extruder device, and that the distal end of the tubular coating is arranged proximally at a distance from the distal end of the stent (1).

9. An aggregate according to any one of the preceding claims, **characterized in that** the length of the stent (1) is reduced at the expansion from the first to the second condition.

10. An aggregate according to any one of claims 1-6, 8, 9, **characterized in that** the tubular coating (2) is arranged in a folded condition around the introducer sheath (3), and that the surfaces facing the introducer sheath are at least partially in contact with an adhesive (15).

11. An endovascular graft device comprising at least a first stent for fixation against a vessel wall in the vascular system and a second stent for placement fully or partially inside the first stent as well as a tubular coating, said first and said second stent having a first condition with a reduced diameter and a expanded second condition with a larger diameter, **characterized in that** at least the major part of the tubular coating (2) is positioned in an aggregate separate from the periphery of the second stent while this stent (1) is in said first condition, that in said second condition the first and the second stents (1, 20) have substantially the same diameter, and that after placement of the first and second stents in said second condition the tubular coating (2) is at least partially fixed between the inner surface of the first stent and the outer surface of the second stent.

## Patentansprüche

1. Aggregat zur transluminalen Einführung eines röhrenförmigen Stents, der in seinem in einem Blutgefäß im vaskulären System angebrachten Zustand an seinem Rand entlang mindestens eines Teils seiner Länge mit einem röhrenförmigen Überzug versehen ist, wobei das Aggregat eine Einführungshülle (3) mit einem Stent (1) umfasst, der im Inneren der Hülle in einem ersten Zustand mit einem verringerten Durchmesser angeordnet ist, sowie eine Abwurfvorrichtung (4), die so gehandhabt werden kann, dass sie den Stent aus der Einführungshülle ablöst, wobei der Stent dafür geeignet ist, sich in einen zweiten Zustand mit einem größeren Durchmesser auszudehnen, **dadurch gekennzeichnet, dass** der Stent (1) in seinem ersten Zustand zumindest teilweise im Inneren der Einführungshülle (3) in direktem Kontakt mit der Innenfläche der Hülle angeordnet ist, und dass zumindest der größere Abschnitt des röhrenförmigen Überzugs (2) getrennt vom Rand des Stents angebracht ist, während sich der Stent in seinem ersten Zustand befindet.

2. Aggregat gemäß Anspruch 1, **dadurch gekennzeichnet, dass** der röhrenförmige Überzug (2) mit dem Stent (1) vorzugsweise an der Stelle auf der Länge des Stents verbunden ist, die sich am oder in nächster Nähe des distalen Endes der Einführungshülle (3) befindet.

3. Aggregat gemäß Anspruch 1, **dadurch gekennzeichnet, dass** das Aggregat den röhrenförmigen Überzug (2) vollständig getrennt vom Stent (1) hält, bis das Ablösen des letzteren begonnen hat.

4. Aggregat gemäß einem der Ansprüche 1 - 3, **dadurch gekennzeichnet, dass** der röhrenförmige Überzug (2) die Einführungshülle (3) umgibt.

5. Aggregat gemäß Anspruch 4, **dadurch gekennzeichnet, dass** das Aggregat einen Katheter (5) umfasst, der den röhrenförmigen Überzug (2) und die Einführungshülle (3) umgibt, vorzugsweise bis das Aggregat transluminal in eine Position vorgeschoben wurde, die sich in der Nähe der Anbringungsstelle befindet.

6. Aggregat gemäß einem der Ansprüche 1 - 5, **dadurch gekennzeichnet, dass** das Aggregat einen ringförmigen Hohlraum (8, 10) umfasst, in dem der röhrenförmige Überzug (2) bis zum Ablösen des Stents geschützt liegt.

7. Aggregat gemäß Anspruch 6, **dadurch gekennzeichnet, dass** der Hohlraum (8) im distalen Ende der Einführungshülle (3) gebildet ist, vorzugsweise indem die Hülle an diesem Ende eine verlängerte Vertiefung aufweist, die einwärts mit Hilfe einer Röhre (9) aus einem elastischen und im Wesentlichen nicht dehnbaren Material geschützt ist.

8. Aggregat gemäß einem der Ansprüche 1 - 7, **dadurch gekennzeichnet, dass** der Stent (1) um die Abwurfvorrichtung (4) herum angeordnet ist, die sich mittig durch die Einführungshülle (3) erstreckt, dass der Stent in seinem ersten Zustand teilweise in dem distalen Abschnitt der Einführungshülle und teilweise in einem ringförmingen Hohlraum (10) im Kopf der Abwurfvorrichtung angeordnet ist, und dass das distale Ende des röhrenförmigen Überzugs proximal in einem Abstand vom distalen Ende des Stents (1) angeordnet ist.

9. Aggregat gemäß einem der vorhergehenden Ansprüche, **dadurch gekennzeichnet, dass** die Länge des Stents (1) bei der Ausdehnung vom ersten in den zweiten Zustand verringert wird.

10. Aggregat gemäß einem der Ansprüche 1, 6, 8, 9, **dadurch gekennzeichnet, dass** der röhrenförmige Überzug (2) in einem gefalteten Zustand um die Einführungshülle (3) angeordnet ist und dass die Oberflächen, die der Einführungshülle (3) zugewandt sind, zumindest teilweise in Kontakt mit einem Klebstoff (15) sind.

11. Endovaskuläre Graftvorrichtung, die mindestens einen ersten Stent zur Befestigung an einer Gefäßwand im vaskulären System und einen zweiten Stent zur vollständigen oder teilweisen Anbringung im Inneren des ersten Stents sowie einen röhrenförmigen Überzug umfasst, wobei der erste und der zweite Stent einen ersten Zustand mit einem verringerten Durchmesser und einen expandierten zweiten Zustand mit einem größeren Durchmesser aufweisen, **dadurch gekennzeichnet, dass** zumindest der größere Abschnitt des röhrenförmigen Überzugs (2) in einem Aggregat getrennt vom Rand des zweiten Stents angeordnet ist, während dieser Stent (1) sich in dem ersten Zustand befindet, dass in dem zweiten Zustand der erste und der zweite Stent (1, 20) im Wesentlichen denselben Durchmesser aufweisen und dass nach der Anbringung des ersten und des zweiten Stents in dem zweiten Zustand der röhrenförmige Überzug (2) zumindest teilweise zwischen der inneren Oberfläche des ersten Stents und der äußeren Oberfläche des zweiten Stents befestigt ist.

## Revendications

1. Ensemble pour l'insertion transluminale d'un extenseur tubulaire qui, dans sa condition en place dans un vaisseau du système vasculaire, est pourvu à sa périphérie d'un revêtement tubulaire le long d'au moins une partie de sa longueur, ledit ensemble incluant une gaine d'introduction (3) avec un extenseur (1) agencé à l'intérieur de la gaine dans une première condition avec un diamètre réduit, et un dispositif d'extraction (4) qui peut être manipulé pour relâcher l'extenseur depuis la gaine d'introduction, l'extenseur étant adapté à se détendre vers une deuxième condition avec un grand diamètre, **caractérisé en ce que** l'extenseur (1) est agencé dans sa première condition au moins partiellement à l'intérieur de la gaine d'introduction (3) en contact direct avec la surface intérieure de la gaine, et **en ce que** la majeure partie au moins du revêtement tubulaire (2) est positionnée de façon séparée de la périphérie de l'extenseur tandis que l'extenseur est dans sa première condition.

2. Ensemble selon la revendication 1, **caractérisé en ce que** le revêtement tubulaire (2) est connecté avec l'extenseur (1), de préférence à l'emplacement sur la longueur de l'extenseur qui se trouve à l'extrémité distale, ou au plus proche de cette extrémité distale de la gaine d'introduction (3).

3. Ensemble selon la revendication 1, **caractérisé en ce que** l'ensemble maintient le revêtement tubulaire (2) complètement séparé de l'extenseur (1) jusqu'à ce que le relâchement de ce dernier a commencé.

4. Ensemble selon l'une quelconque des revendications 1 à 3, **caractérisé en ce que** le revêtement tubulaire (2) entoure la gaine d'introduction (3).

5. Ensemble selon la revendication 4, **caractérisé en ce que** l'ensemble comprend un cathéter (5) qui entoure le revêtement tubulaire (2) et la gaine d'introduction (3), de préférence jusqu'à ce que l'ensemble a été avancé par voie transluminale jusqu'à un emplacement proche du site de mise en place.

6. Ensemble selon l'une quelconque des revendications 1 à 5, **caractérisé en ce que** l'ensemble comprend une cavité annulaire (8, 10) dans laquelle le revêtement tubulaire (2) repose de façon protégée jusqu'au relâchement de l'extenseur.

7. Ensemble selon la revendication 6, **caractérisé en ce que** la cavité (8) est formée dans l'extrémité distale de la gaine d'introduction (3), de préférence du fait que la gaine comporte au niveau de cette extrémité un évidement allongé, protégé vers l'intérieur au moyen d'un tube (9) d'un matériau flexible et sensiblement non étirable.

8. Ensemble selon l'une quelconque des revendications 1 à 7, **caractérisé en ce que** l'extenseur (1) est agencé autour du dispositif d'extraction (4) en s'étendant au centre à travers la gaine d'introduction (3), **en ce que** l'extenseur est situé dans sa première condition partiellement dans le tronçon distal de la gaine d'introduction et partiellement dans une cavité annulaire (10) dans la tête du dispositif d'extraction, et **en ce que** l'extrémité distale du revêtement tubulaire est agencée de façon proximale à une distance de l'extrémité distale de l'extenseur (1).

9. Ensemble selon l'une quelconque des revendications précédentes, **caractérisé en ce que** la longueur de l'extenseur (1) est réduite lors de l'expansion de la première à la deuxième condition.

10. Ensemble selon l'une quelconque des revendications 1 à 6,8 et 9, **caractérisé en ce que** le revêtement tubulaire (2) est agencé dans une condition repliée autour de la gaine d'introduction (3), et **en ce que** les surfaces qui font face à la gaine d'introduction sont au moins partiellement en contact avec un adhésif (15).

11. Dispositif de greffage endovasculaire comprenant au moins un premier extenseur destiné à être fixé contre une paroi d'un vaisseau du système vasculaire, et un deuxième extenseur destiné à être placé totalement ou partiellement à l'intérieur du premier extenseur, ainsi qu'un revêtement tubulaire, ledit premier et ledit deuxième extenseur ayant une première condition avec un diamètre réduit et une deuxième condition dilatée avec un grand diamètre, **caractérisé en ce que** la majeure partie au moins du revêtement tubulaire (2) est positionnée dans un ensemble séparé de la périphérie du deuxième extenseur tandis que cet extenseur (1) est dans ladite première condition, **en ce que** dans ladite deuxième condition le premier extenseur et le deuxième extenseur (1, 20) ont sensiblement le même diamètre, et **en ce qu'**après mise en place du premier extenseur et du deuxième extenseur dans la ladite deuxième condition, le revêtement tubulaire (2) est au moins partiellement fixé entre la surface intérieure du premier extenseur et la surface extérieure du deuxième extenseur.
